(19) **Europäisches Patentamt**
**European Patent Office**
**Office européen des brevets**

(11) **EP 2 151 682 B1**

(12) **EUROPEAN PATENT SPECIFICATION**

(45) Date of publication and mention
of the grant of the patent:
**17.09.2014 Bulletin 2014/38**

(51) Int Cl.:
**G01N 27/26** *(2006.01)* **G01N 27/416** *(2006.01)*
**G01N 30/64** *(2006.01)* **G01N 30/88** *(2006.01)*
**G01N 27/327** *(2006.01)*

(21) Application number: **08740891.0**

(22) Date of filing: **25.04.2008**

(86) International application number:
**PCT/JP2008/058152**

(87) International publication number:
**WO 2008/133332 (06.11.2008 Gazette 2008/45)**

(54) **SUBSTRATE CONCENTRATION MEASUREMENT METHOD AND SUBSTRATE CONCENTRATION MEASUREMENT APPARATUS**

SUBSTRATKONZENTRATIONSMESSVERFAHREN UND
SUBSTRATKONZENTRATIONSMESSVORRICHTUNG

PROCÉDÉ ET APPAREIL DE MESURE DE CONCENTRATION DE SUBSTRAT

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **25.04.2007 JP 2007116099**

(43) Date of publication of application:
**10.02.2010 Bulletin 2010/06**

(73) Proprietor: **ARKRAY, Inc.**
**Kyoto-shi, Kyoto 601-8045 (JP)**

(72) Inventors:
• **KAMATA, Tatsuo**
**Kyoto-shi**
**Kyoto 601-8045 (JP)**

• **TAKAGI, Takeshi**
**Kyoto-shi**
**Kyoto 601-8045 (JP)**
• **ITO, Yuki**
**Kyoto-shi**
**Kyoto 601-8045 (JP)**

(74) Representative: **HOFFMANN EITLE**
**Patent- und Rechtsanwälte**
**Arabellastrasse 4**
**81925 München (DE)**

(56) References cited:
WO-A1-2004/113913 WO-A1-2005/098424
WO-A1-2005/098424 WO-A1-2005/103669
JP-A- 02 159 563 JP-A- 2000 346 848

EP 2 151 682 B1

**Description**

TECHNICAL FIELD

The present invention relates to a method and an apparatus for measuring a concentration of a substrate such as glucose in a specimen containing hemoglobin.

BACKGROUND TECHNOLOGY

As a method for measuring a concentration of a substrate such as glucose, an electrode method has been known. The method includes causing an electrode contacting a specimen to output information correlating with a substrate concentration in the specimen and calculating the substrate concentration based on the output. The electrode method can be divided broadly into an equilibrium position method (end point method) and a differentiation method (rate method). The equilibrium position method is employed for calculating a glucose concentration based on an equilibrium value when a temporal change of the output from the electrode approaches to a constant value. In turn, the differentiation method is employed for calculating a glucose concentration based on a maximum value when the output is differentiated for n times (n is a positive integer).

In the general electrode method, it has been known that the measurement value is decreased due to the influence of blood cells in the case of using whole blood as the specimen. Therefore, there has been proposed a method of relating a calculation result (EP value) in the equilibrium position method with a calculation result (DI value) in the differentiation method in the case of measuring the glucose concentration in blood (see Patent Documents 1 and 2, for example).

The methods disclosed in Patent Documents 1 and 2 take a total amount of glucose diffused out of blood cells until a detection of an output corresponding to the maximum value and a speed of the diffusion into consideration in the case of calculating a glucose concentration in the differentiation method. Since these methods correlate differences in total amount and diffusion speed of glucose diffused out of blood cells with a difference between the EP value and the DI value to correct the influence of blood cells, the existence of blood cells in the specimen is the premise of the method.

Meanwhile, in the case of using a hemolysate specimen as a sample or in the case of measuring after diluting a sample with a diluent liquid containing a reagent that hemolyzes blood cells, hemoglobin is contained in the specimen. Also, hemoglobin is contained in the specimen when blood cells are hemolyzed in the case of using whole blood or when blood cells are hemolyzed before centrifugation in the case of using a serum or plasma.

Patent Document 3 describes a biosensor system, method and apparatus for implementing threshold based correction functions for biosensors. In there, a primary measurement of an analyte value is obtained. A second measurement of a secondary effect is obtained and is compared with a threshold value. A correction function is identified responsive to the compared values. The correction function is further applied to primary measurement of the analyte value to provide a corrected analyte value. In addition, the correction method uses correction curves that are provided to correct for interference effects.

   Patent Document 1: Japanese Unexamined Patent Publication No.9-33533
   Patent Document 2: Japanese Patent Publication No.9-318634
   Patent Document 3: WO 2005/098424 A

DISCLOSURE OF THE INVENTION PROBLEMS TO

BE SOLVED BY THE INVENTION

However, in the case of measuring a glucose concentration or the like, a measurement value tends to be decreased due to hemoglobin contained in the specimen. Particularly, in the case of measuring a substrate concentration by using a hydrogen peroxide electrode, an output from the electrode tends to be decreased by a degree of the reaction between hemoglobin and hydrogen peroxide since hemoglobin easily reacts with hydrogen peroxide, and, therefore, it is difficult to achieve high measurement accuracy.

Though it is not possible to avoid hemoglobin from being contained in the specimen by preventing hemolysis of blood cells, such method is not realistic since the method requires extreme caution for transport/storage and pretreatment of blood and incurs not only complicated work but also a variation in hemolysis of samples.

An object of the present invention is to improve measurement accuracy by suppressing the influence of hemoglobin in measurement of a concentration of a substrate such as glucose in a specimen such as blood without any complicated work.

MEANS FOR SOLVING THE PROBLEM

According to a first aspect of the present invention, there is provided a substrate concentration measurement method including measuring a concentration of a substrate in a blood specimen containing hemoglobin, wherein the substrate concentration is calculated by using a measurement value correlating with a substrate concentration influenced by hemoglobin and a hemoglobin concentration or a value correlating with the hemoglobin concentration.

According to the present invention, the substrate concentration is calculated by correcting the measurement value correlating with the substrate concentration based on the following Formulas 1 and 2.

$$[\text{Formula 1}]$$

$$\text{Corrected value} = 100 \times Re/V\ (\%),$$

wherein
V (%) represents a value with respect to plasma (a ratio of a measurement value influenced by hemoglobin with respect to a measurement value obtained by measuring plasma); and
Re represents a measurement value correlating with a substrate concentration influenced by hemoglobin.

$$[\text{Formula 2}]$$

$$\text{Value with respect to plasma V}\ (\%) = (Vmax \times Re)/(Km + Re) + B,$$

wherein

Vmax represents a value obtained by subtracting an intercept (B) from a value (V) with respect to plasma which is maintained constant even when a measurement value influenced by hemoglobin increases;
Km represents a value of Re at which a value V (%) with respect to plasma becomes Vmax/2 + B and
B represents a value (constant) of V (%) with respect to plasma when Re is O.

Vmax may preferably be determined based on a measurement result of the hemoglobin concentration or the value correlating with the hemoglobin concentration of the blood specimen and, for example, is determined based on a chromatogram obtainable by chromatography.

Re may preferably be measured by employing an enzyme electrode method and, for example, is detected as a voltage value or a current value.

According to a second aspect of the present invention, there is provided a substrate concentration measurement apparatus including calculation means for calculating a concentration of a substrate in a blood specimen containing hemoglobin and substrate measurement means for measuring a value correlating with a substrate concentration influenced by hemoglobin, wherein the substrate concentration measurement apparatus further includes hemoglobin measurement means for measuring a hemoglobin concentration or a value correlating with the hemoglobin concentration, and the calculation means calculates the substrate concentration by using the measurement value in the substrate measurement means and the measurement value in the hemoglobin measurement means.

The calculation means is adapted to calculate the substrate concentration by correcting the measurement value Re correlating with the substrate concentration based on the following Formulas 1 and 2.

$$[\text{Formula 1}]$$

$$\text{Corrected value} = 100 \times Re/V\ (\%),$$

wherein
V (%) represents a value with respect to plasma (a ratio of a measurement value influenced by hemoglobin with respect to a measurement value obtained by measuring plasma); and
Re represents a measurement value correlating with a substrate concentration influenced by hemoglobin.

[Formula 2]

$$\text{Value with respect to plasma } V \ (\%) = (V_{max} \times Re)/(Km + Re) + B,$$

wherein

Vmax represents a value obtained by subtracting an intercept (B) from a value (V) with respect to plasma which is maintained constant even when a measurement value influenced by hemoglobin increases;

Km represents a value of Re at which a value V (%) with respect to plasma becomes Vmax/2 + B and

B represents a value (constant) of V (%) with respect to plasma when Re is 0.

The calculation means is adapted to determine Vmax by measuring the hemoglobin concentration or the value correlating with the hemoglobin concentration of the blood specimen

The hemoglobin measurement means is adapted to measure the hemoglobin concentration or the value correlating with the hemoglobin concentration as a chromatogram obtainable by chromatography.

The substrate measurement means is adapted to be provided with an enzyme electrode for obtaining the measurement value Re correlating with the substrate concentration influenced by hemoglobin, for example. In this case, the measurement value Re is a current value outputted in the enzyme electrode or a voltage value obtained by converting the current value.

The blood specimen that is the object for measurement in the present invention is whole blood or a diluted liquid thereof, for example, and the substrate is glucose, for example.

BRIEF DESCRIPTION OF THE DRAWINGS

Fig. 1 is a schematic diagram showing a schematic structure of a substrate concentration measurement apparatus according to the present invention.

Fig. 2 is a diagram showing one example of chromatogram obtained in a hemoglobin measurement unit in the substrate concentration measurement apparatus shown n Fig. 1.

Fig. 3 is a sectional view showing a schematic structure of an enzyme electrode in the substrate concentration measurement unit in the substrate concentration measurement apparatus shown in Fig. 1.

Fig. 4 is a block diagram showing the substrate concentration measurement apparatus shown in Fig. 1.

Fig. 5 is a graph schematically showing a relationship between an output (voltage value) and a value with respect to plasma in the enzyme electrode.

Fig. 6 is a graph schematically showing a relationship between Vmax and a hemoglobin amount.

Fig. 7 is a graph showing a relationship between an output (voltage value) and a value with respect to plasma in the enzyme electrode when a hematocrit value is 45% in Example 1.

Fig. 8 is a graph showing a relationship between an output (voltage value) and a value with respect to plasma in the enzyme electrode when a hematocrit value is 35% in Example 1.

Fig. 9 is a graph showing a relationship between an output (voltage value) and a value with respect to plasma in the enzyme electrode when a hematocrit value is 60% in Example 1.

Fig. 10 is a graph showing accuracy of a corrected value in Example 1.

DESCRIPTION OF REFERENCE SYMBOLS

1    substrate concentration measurement apparatus
2    hemoglobin measurement unit (hemoglobin measurement means)
3    substrate measurement unit (substrate measurement means)
34   enzyme electrode
5    calculation section (calculation means)

PREFERRED EMBODIMENT OF THE INVENTION

Hereinafter, the present invention will be described with reference to the drawings.

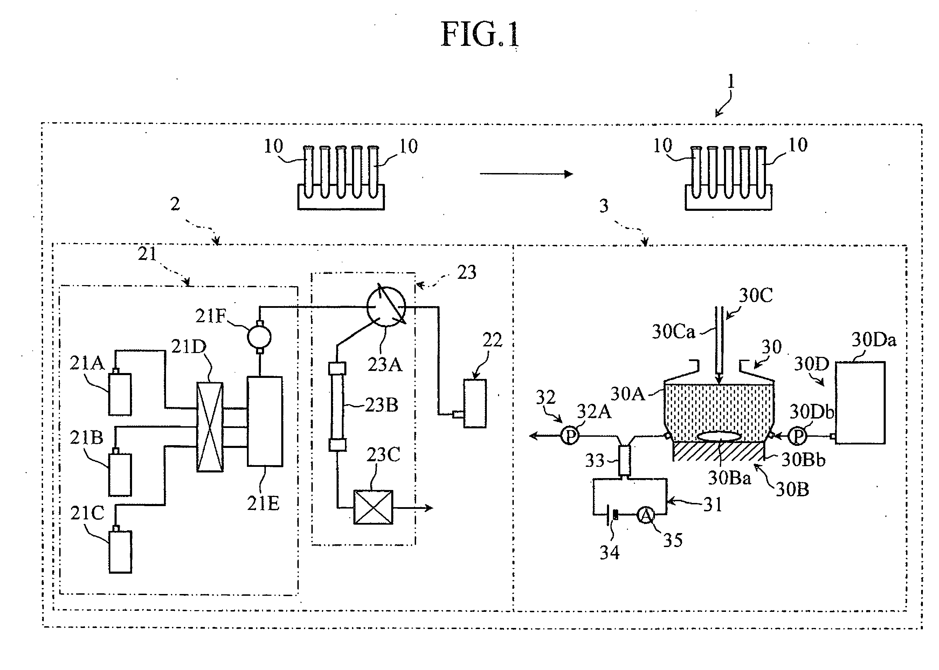

A concentration measurement apparatus 1 shown in Fig. 1 is adapted to be capable of measuring a hemoglobin amount and a substrate concentration in a blood specimen and is provided with a hemoglobin measurement unit 2 and a substrate measurement unit 3.

The hemoglobin measurement unit 2 is adapted to be capable of measuring the hemoglobin amount by way of liquid chromatography and is provided with a liquid supply mechanism 21, a specimen preparation mechanism 22, and a hemoglobin measurement mechanism 23.

The liquid supply mechanism 21 serves to supply an elution liquid or a washing hemolyzing liquid of a bottle 21A, 21B, or 21C independently to an injection valve 23A of the hemoglobin measurement mechanism 23 described later in this specification. The liquid supply mechanism 21 includes a degassing device 21D, a manifold 21E, and a pump 21F in addition to the bottles 21A, 21B, and 21C.

The bottles 21A and 21B serves to retain the elution liquids to be supplied to an analysis column 23B in the hemoglobin measurement mechanism 23 described later in this specification. As the elution liquid, buffers having varied pH values or salt concentrations are used. The bottle 21C serves to retain the hemolyzing washing liquid. The hemolyzing washing liquid has both of capability of hemolyzing blood cells in whole blood and diluting a target component and a capability of washing pipings. The hemolyzing washing liquid includes a buffering agent and a hemolyzing agent, and a supporting electrolyte such as NaCl and a preservative such as Na azide may be added to the hemolyzing washing liquid as needed.

The degassing device 21D serves to eliminate a dissolved gas from the elution liquids before supplying the elution liquids and the hemolyzing washing liquid to the analysis column 23B described later in this specification. It is possible to form the degassing device 21D by forming a part of a flow path of each of the elution liquids by a hollow gas-liquid separation film and disposing the gas-liquid separation film in a chamber. With such a structure, it is possible to eliminate the dissolved gas from the elution liquids circulating inside the gas-liquid separation film by depressurizing the chamber. Needless to say, a structure other than the structure of depressurizing the chamber housing the gas-liquid separation film may be used as the degassing device 21D.

The manifold 21E serves to select a type of liquid (type of the bottle 21A, 21B, or 21C) to be supplied to the injection valve 23A and controlled by control means outside the drawing.

The pump 21F serves to supply the liquids of the bottles 21A, 21B, and 21C to the injection valve 23A and the analysis column 23B described later in this specification. As the pump 21F, known various pumps are usable.

The specimen preparation mechanism 22 serves to prepare a measurement specimen to be introduced into the hemoglobin measurement mechanism 23. The specimen preparation mechanism 22 is adapted to prepare the measurement specimen by supplying a sample such as blood retained in a blood collection tube 10 and a diluent liquid to a dilution tank by using nozzles.

The hemoglobin measurement mechanism 23 serves to measure a hemoglobin amount in the specimen by employing a liquid chromatography method and includes the injection valve 23A, the analysis column 23B, and a photometer unit 23C.

The injection valve 23A determines a certain amount (several microliters, for example) of the specimen to be introduced into the analysis column 23B and allows the specimen to be introduced into the analysis column 23B. The injection valve 23A is connected to the specimen preparation mechanism 22, the analysis column 23B, and a waste liquid tank (not shown).

The analysis column 23B is filled with a filler and maintained to a target temperature of about 40°C, for example, by a temperature regulation mechanism (not shown). Here, in the case of measuring a concentration of hemoglobin, a methacrylic ester copolymer, for example, is used as the filler.

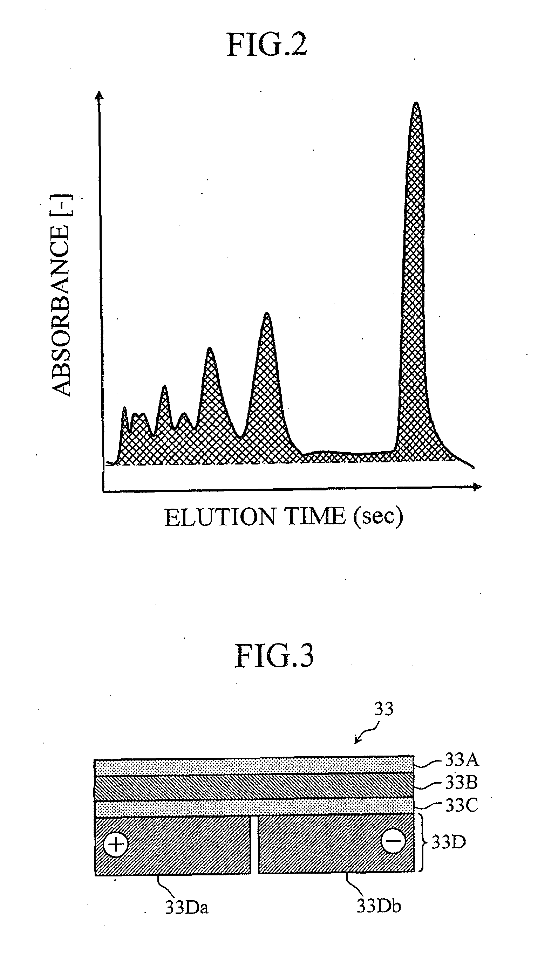

The photometer unit 23C serves to optically detect hemoglobin contained in a desorption liquid from the analysis column 23B and is adapted to obtain an output (absorbance, for example) responsive to a hemoglobin amount. The output corresponding to the hemoglobin amount is obtained as the chromatogram shown in Fig. 2, for example. From the chromatogram, it is possible to figure out a total hemoglobin amount and a glycohemoglobin concentration (a ratio of glycohemoglobin with respect to the total hemoglobin amount).

As shown in Fig. 1, the substrate measurement unit 3 has the specimen preparation mechanism 30 and a detection mechanism 31.

The specimen preparation mechanism 30 serves to prepare a specimen by diluting a sample and includes a preparation vessel 30A, a stirring operation section 30B, a sample supply section 30C, and a diluent liquid supply section 30D.

The preparation vessel 30A serves to provide a place for preparing a specimen by diluting a sample.

The stirring operation section 30B stirs and mixes a specimen and a reagent when the specimen and the reagent are supplied to the preparation vessel 30A and includes a stirring bar 30Ba and a stirrer 30Bb. The stirring bar 30Ba is housed in the preparation vessel 30A, and a liquid in the preparation vessel 30A is stirred when the stirring bar 30Ba is rotated by the stirrer 30Bb.

The specimen supply section 30C serves to supply a sample such as blood retained in the blood collection tube 10 to the preparation vessel 30A and has a nozzle 30Ca. As the sample, a blood specimen (a diluted liquid of whole blood, plasma, or serum) is used.

The diluent liquid supply section 30D serves to supply the diluent liquid to the preparation vessel 30A and has a diluent liquid tank 30Da and a pump 30Db. The diluent liquid tank 30Da retains the diluent liquid to be supplied to the preparation vessel 30A. As the diluent liquid, a buffer solution is used, for example, and a supporting electrolyte such as NaCl and a preservative such as Na azide may be added to the diluent liquid as needed. The pump 30Db serves to impart power for supplying the diluent liquid of the diluent liquid tank 30Da to the preparation vessel 30A.

The detection mechanism 31 is provided with the enzyme electrode 33, a power source 34, and a current value measurement section 35.

The enzyme electrode 33 outputs an electrical physical amount responsive to an amount of electrons given/received to/from a substrate in the sample. The enzyme electrode 33 has a substrate permselective film 33A, an enzyme immobilized film 33B, a hydrogen peroxide permselective film 33C, and a hydrogen peroxide electrode 33.

The substrate permselective film 33A serves to selectively donate a substrate in a sample to the enzyme immobilized layer 33B. The substrate permselective film 33A is chosen depending on a type of the substrate, and known various substrate permselective films are usable as the substrate permselective film 33A.

The enzyme immobilized film 33B serves to generate hydrogen peroxide by oxidizing a substrate and structured to contain oxidase. The oxidase to be used in the present invention is chosen depending on a type of the substrate. Here, glucose or lactate may be used as the substrate serving as the object for measurement in the substrate measurement unit 3, and glucose oxidase or lactate oxidase may be used as the oxidase.

The hydrogen peroxide permselective film 33C serves to selectively donate hydrogen peroxide to the hydrogen peroxide electrode 33D. As the hydrogen peroxide permselective film 33C, an acetylcellulose-based or polyallylamine-based film may be used.

The hydrogen peroxide electrode 33D outputs an electric signal responsive to an amount of donated hydrogen peroxide, i.e., to a concentration of a substrate. As the hydrogen peroxide electrode 33D, an electrode using platinum for an anode 33Da and silver for a cathode 33Db may be used.

The power source 34 shown Fig. 1 serves to apply a voltage to the enzyme electrode 33 (hydrogen peroxide electrode 33D). As the power source 34, a direct current power source is used, for example, and an applied voltage to the enzyme electrode 33 (hydrogen peroxide electrode 33D) is set to 100 to 500 mV, for example.

The current value measurement section 35 serves to measure an amount of electrons given/received between the hydrogen peroxide electrode 33D and hydrogen peroxide as a current value. The measurement of the current value in the current value measurement section 35 is intermittently performed.

A drainage mechanism 32 serves to discard a liquid existing in the preparation vessel 30A after the measurement and has a pump 32A. With the drainage mechanism 32, the liquid in the preparation vessel 30A is discharged by power from the pump 32A.

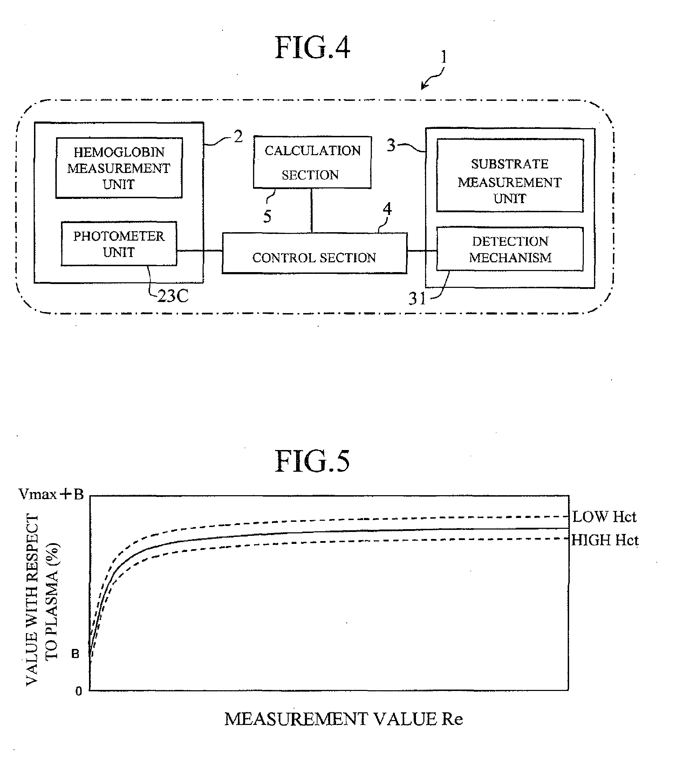

As shown in Fig. 4, the substrate concentration measurement apparatus 1 is further provided with a control section 4 and a calculation section 5.

The control section 4 serves to control operations of the sections. More specifically, the control section 4 controls the operations of the manifold 21E, the pump 21F, the injection valve 23A, the photometer unit 23C, and the like in the hemoglobin measurement unit 2 as well as the operations of the pumps 30Db and 32A, the nozzle 30Ca, the stirrer 30Bb, and the like in the substrate measurement unit 3. The control section 4 further controls the operation of the detection mechanism 31 in the substrate measurement unit 3. More specifically, the control section 4 selects between a state in which the voltage is applied to the hydrogen peroxide electrode 33D and a state in which the voltage is not applied to the hydrogen peroxide electrode 33D by controlling the power source 34 shown in Fig. 1 to control the current value measurement section 35, thereby controlling a timing for the current value measurement. The measurement operation of the current value measurement section 35 is controlled by the control section 4 in such a manner that the current value is measured repeatedly at an interval of 50 to 200 μsec, for example.

The operation section 5 serves to calculate a substrate concentration of glucose or the like in a sample based on the measurement results in the photometer unit 23C of the hemoglobin measurement unit 2 and the current value measurement section 35 (see Fig. 1) of the substrate measurement unit 3. The calculation section 5 stores a program required for the calculation, and the operation thereof is controlled by the control section 4. In the present embodiment, the calculation section 5 is structured to calculate the substrate concentration based on a corrected value that is obtained by correcting the output in the current value measurement section 35 by taking the hemoglobin concentration into consideration. The output from the current value measurement section 35 may be corrected as a current value, but it is preferable to perform the correction after converting the current value into a voltage value.

In the calculation section 5, a voltage value obtained by converting a current value obtained in the current value measurement section 35 in accordance with a certain conversion formula, for example, is corrected based on the following Formula 1.

[Formula 1]

$$\text{Corrected value} = 100 \times Re/V \ (\%),$$

wherein
V (%) represents a value with respect to plasma (a ratio of a measurement value influenced by hemoglobin with respect to a measurement value obtained by measuring plasma); and
Re represents a measurement value correlating with a substrate concentration influenced by hemoglobin.
In Formula 1, the value V (%) with respect to plasma is a value obtained by the following Formula 2.

[Formula 2]

$$\text{Value with respect to plasma } V \ (\%) = (Vmax \times Re)/(Km+Re)+B,$$

wherein

Vmax represents a value obtained by subtracting an intercept (B) from a value (V) with respect to plasma which is maintained constant even when a measurement value influenced by hemoglobin increases;
Km represents a value of Re at which a value V (%) with respect to plasma becomes Vmax/2 + B and
B represents a value (constant) of V (%) with respect to plasma when Re is 0.

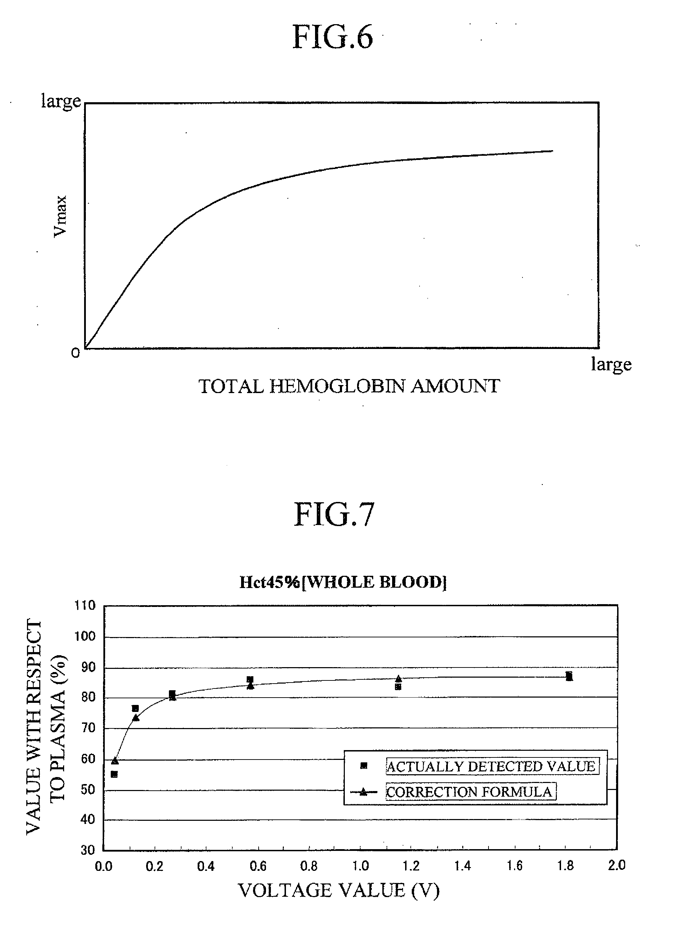

The calculation of substrate concentration in the calculation section 5 is performed by relating the corrected value to a standard curve or a table indicating a correlation between corrected values obtained by Formula 1 and substrate concentrations.
Here, the value with respect to plasma (V (%)) indicates a ratio of a measurement value influenced by hemoglobin with respect to a measurement value obtained by measuring plasma.
As shown in Fig. 5, in the case where the value with respect to plasma (V (%)) is the vertical axis and the measurement value Re correlating with the substrate concentration is the horizontal axis, Formula 2 indicates that the measurement value Re approaches to the constant value (Vmax+B) along with an increase in the measurement value Re when the intercept (intersection with the vertical axis) of the value with respect to plasma (V (%)) of the blood specimen is B. In other words, in the case where a substrate concentration is measured by using a blood specimen, Formula 2 is an approximation formula that detects the tendency that the measurement value is decreased with respect to the plasma value as the decrease in output in the hydrogen peroxide electrode 33D caused by the reaction of a part of hydrogen peroxide generated in the enzyme immobilized film 33B with hemoglobin without consumption of hydrogen peroxide in the hydrogen peroxide electrode 33D. Also, Formula 2 is a relational formula similar to the Michaelis-Menten equation, and it is considered that a speed of transfer of hydrogen peroxide that is generated in the enzyme immobilized film 33B to the hydrogen peroxide electrode 33D is involved in the present invention as a principle that allows adaptation of Michaelis-Menten. That is, the inventors estimate the reduction in speed of transfer of hydrogen peroxide to the hydrogen peroxide electrode 33D which is caused by the increase in hemoglobin as a factor that allows the adaptation of the Michaelis-Menten equation.
The tendency that the measurement value is decreased with respect to the plasma value in the case where a substrate concentration is measured by using a blood specimen can be detected also as a decrease in ratio of solubilized glucose caused by the influence of a solid component (hematocrit value) in the blood specimen. That is, as shown in Fig. 5, in the comparison between the case wherein the hematocrit value is small and the case wherein the hematocrit value is large, since the influence of solid component becomes relatively small when the hematocrit value is small, the value with respect to plasma is increased when the hematocrit value is small, and, in contrast, there is a tendency that the value with respect to plasma is decreased when the hematocrit value is large. In order to take the influence of hematocrit value into consideration, it is preferable to derive Vmax from the hemoglobin concentration or the value correlating to the hemoglobin concentration.
Here, since Vmax in Formula 2 is corresponding to the plasma value of the blood specimen, Vmax has a tendency of being increased along with an increase in hemoglobin amount, but, the plasma value is also influenced by the solid component (hematocrit value). Therefore, as shown in Fig. 6, since the influence of hemoglobin is increased due to the increase in hemoglobin amount in blood specimen that is caused by the increase in hematocrit value, there is a tendency that Vmax approaches to the constant value. Therefore, it is possible to correlate Vmax with the hemoglobin amount, and, for example, in the case of figuring out the hemoglobin amount from the chromatogram obtained by chromatography, it is possible to determine Vmax from a total area of hemoglobin in the chromatogram (part of cross hatching in Fig. 2).

Meanwhile, it is possible to set the constant B in Formula 2 by measuring a target substrate by using a plurality of samples of healthy subjects and from a curvature of Formula 2 that fits the measurement values.

In the present invention, a final substrate concentration is calculated by taking the influence of the amount of hemoglobin contained in the blood specimen into consideration. Therefore, in the present invention, it is possible to perform calculation of the substrate concentration by taking the decrease caused by the reaction of hemoglobin with hydrogen peroxide generated in the enzyme immobilized film 33B into consideration. As a result, the present invention enables the measurement of substrate concentration in blood specimen with excellent accuracy.

Further, by reflecting the influence of the hemoglobin amount to Vmax, it is possible to take the influence of the solid component (hematocrit value) in the blood specimen also into consideration. Therefore, in the present invention, it is possible to perform calculation of the substrate concentration by taking the decrease in ratio of solubilized glucose caused by the influence of solid component (hematocrit value) in blood specimen into consideration. As a result, the present invention enables the measurement of substrate concentration in blood specimen with much more excellent accuracy.

It is possible to modify the present invention into various modes without limitation to the above-described embodiment. In other words, the gist of the present invention lies in the feature of taking the influence of hemoglobin in blood specimen into consideration. The output of the hydrogen peroxide electrode 33D is not necessarily corrected by the correction formula indicated as Formula 1, and another correction formula may be used for taking the influence of hemoglobin into consideration.

Also, it is not always necessary to provide the hemoglobin measurement unit 2 in addition to the substrate measurement unit 3 as in the concentration measurement apparatus 1 shown in Fig. 1, and the hemoglobin measurement unit 2 may be provided separately from the substrate concentration measurement apparatus 1. It is not always necessary to employ the chromatography method as the hemoglobin measurement method.

Hereinafter, the present invention will be described based on Example. The present invention is not limited to Example described below.

EXAMPLE

In Example, as to each of a plurality of blood specimens having varied glucose concentrations, a value with respect to plasma in the case where an output (voltage value) in the substrate measurement unit is corrected by Formula 2 was investigated. The investigation of the value with respect to plasma was conducted on three types of hematocrit values.

In Formula 2, Vmax, Km, and B are set as shown in Table 1 for each of the hematocrit values.

[Table 1]

| Hematocrit value (%) | 45 | 35 | 55 |
| --- | --- | --- | --- |
| Vmax | 55 | 62 | 54 |
| Km | 0.04 | 0.04 | 0.04 |
| B | 30 | 30 | 30 |

A hemoglobin concentration was measured as a total area of hemoglobin in a chromatogram by using "HA-8160" (trade name: product of Arkray, Inc.). The measurement values of the total areas of hemoglobin of the specimens with varied hematocrit values are shown in Table 2 to Table 4.

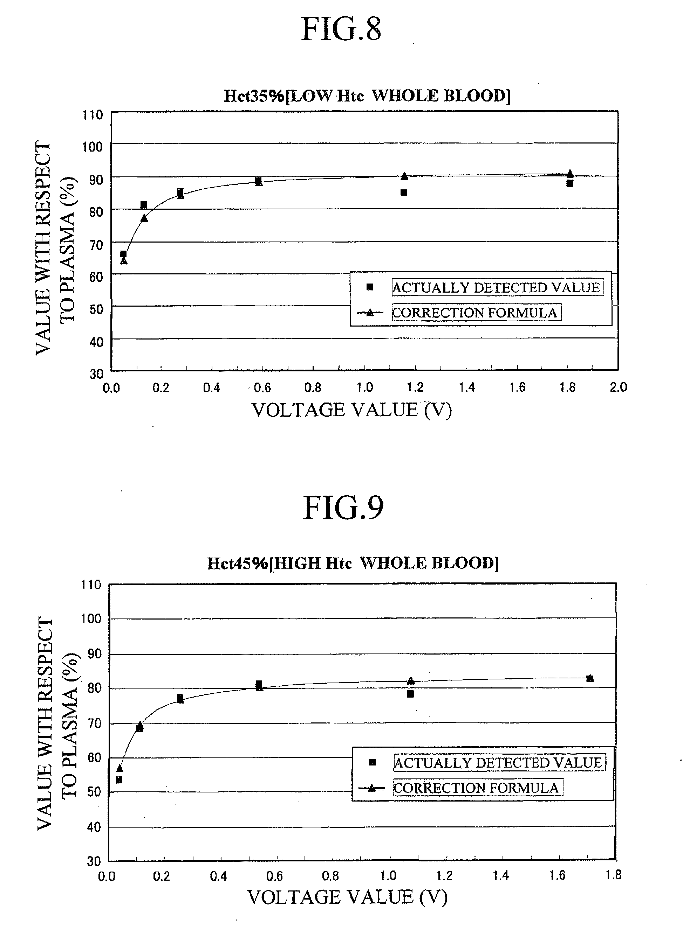

An output correlating with a glucose concentration in each of blood specimens was measured as a voltage value by using "HA-8160" (trade name: product of Arkray, Inc.). The measurement values of the voltage values for the varied hematocrit values are shown in Table 2 to Table 4 together with values Vs (%) with respect to plasma corresponding to the measured voltage values. Further, relationships between the measurement value (voltage value) and the value with respect to plasma are shown in Fig. 7 to Fig. 9.

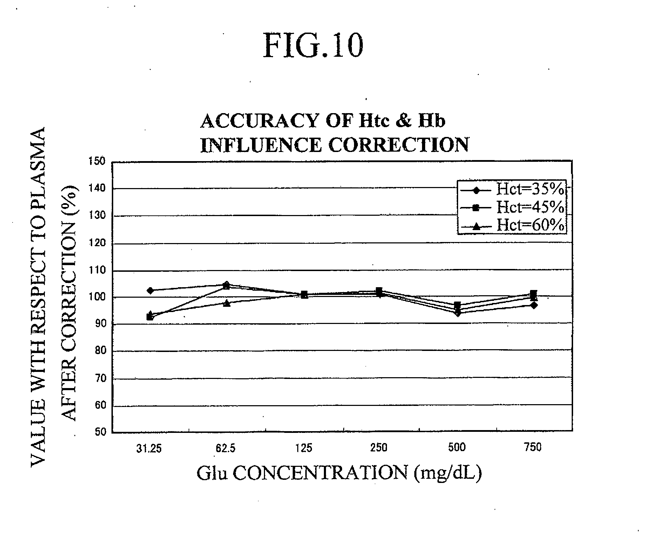

Values Vr (%) with respect to plasma corrected by Formula 2 are shown in Table 2 to Table 4, and a graph showing the glucose concentration on the horizontal axis and the value with respect to plasma (%) on the vertical axis is shown in Fig. 10.

[Table 2]

| Hematocrit value: 45% | | | | | |
|---|---|---|---|---|---|
| Glucose concentration | Hemoglobin total area | Voltage value | Value Vs with respect to plasma actually detected | Value Vr with respect to plasma obtained by Formula 2 | 100 Vs/Vr |
| 31.25 mg/dL | 29516 | 0.0412 V | 54.98 % | 59.43 % | 92.5 % |
| 62.5 mg/dL | 29358 | 0.1228 V | 76.60 % | 73.75 % | 103.9 % |
| 125 mg/dL | 28993 | 0.2682 V | 81.07 % | 80.47 % | 100.7 % |
| 250 mg/dL | 29502 | 0.5662 V | 85.85 % | 84.17 % | 102.0 % |
| 500 mg/dL | 30036 | 1.473 V | 83.38 % | 86.05 % | 96.9 % |
| 700 mg/dL | 29821 | 1.8123 V | 87.40 % | 86.75 % | 100.8 % |

[Table 3]

| Hematocrit value: 35% | | | | | |
|---|---|---|---|---|---|
| Glucose concentration | Hemoglobin total area | Voltage value | Value Vs with respect to plasma actually detected | Value Vr with respect to plasma obtained by Formula 2 | 100 Vs/Vr |
| 31.25 mg/dL | 24527 | 0.0493 V | 65.83 % | 64.24 % | 102.5 % |
| 62.5 mg/dL | 23491 | 0.1298 V | 80.97 % | 77.39 % | 104.6 % |
| 125 mg/dL | 23694 | 0.2807 V | 84.84 % | 84.27 % | 100.7 % |
| 250 mg/dL | 23446 | 0.5845 V | 88.63 % | 88.03 % | 100.7 % |
| 500 mg/dL | 23830 | 1.1625 V | 84.48 % | 89.94 % | 93.9 % |
| 700 mg/dL | 24002 | 1.8151 V | 87.53 % | 90.66 % | 96.5 % |

[Table 4]

| Hematocrit value: 55% | | | | | |
|---|---|---|---|---|---|
| Glucose concentration | Hemoglobin total area | Voltage value | Value Vs with respect to plasma actually detected | Value Vr with respect to plasma obtained by Formula 2 | 100 Vs/Vr |
| 31.25 mg/dL | 37787 | 0.0401 V | 53.47 % | 57.03 % | 93.8 % |
| 62.5 mg/dL | 38578 | 0.1093 V | 68.18 % | 69.53 % | 98.1 % |
| 125 mg/dL | 37713 | 0.2555 V | 77.22 % | 76.69 % | 100.7 % |
| 250 mg/dL | 38478 | 0.5350 V | 81.12 % | 80.24 % | 101.1 % |
| 500 mg/dL | 38329 | 1.0748 V | 78.11 % | 82.06 % | 95.2 % |

(continued)

| Hematocrit value: 55% | | | | | |
|---|---|---|---|---|---|
| Glucose concentration | Hemoglobin total area | Voltage value | Value Vs with respect to plasma actually detected | Value Vr with respect to plasma obtained by Formula 2 | 100 Vs/Vr |
| 700 mg/dL | 39116 | 1.7089 V | 82.41 % | 82.76 % | 99.6 % |

As is apparent from Table 2 to Table 4 and Fig. 7 to Fig. 9, Formula 2 appropriately reflects the relationship between the actually detected voltage value and the value with respect to plasma. Particularly, as is apparent from Fig. 10, the value with respect to plasma calculated by Formula 2 has a small deviation from the actually detected value with respect to plasma over the wide range from a low range to a high range of the glucose concentration. Therefore, according to Formula 2, it is possible to obtain an appropriate value with respect to plasma from an actually detected voltage value. As a result, it is possible to obtain a value from which the influence of hemoglobin is appropriately eliminated by calculating the glucose concentration by correcting the measurement value by Formula 1 based on the value with respect to plasma obtained by Formula 2.

Also, even when the hematocrit value is varied, Formula 2 appropriately reflects the relationship between the actually detected voltage value and the value with respect to plasma irrespective of a size of the hematocrit value. Therefore, it is possible to obtain a value from which the influence of the solid component (hematocrit value) is appropriately eliminated by calculating the glucose concentration by correcting the measurement value by the correction formula such as Formula 1 based on the value with respect to plasma obtained by Formula 2.

**Claims**

1. A substrate concentration measurement method for measuring a concentration of a substrate in a blood specimen containing hemoglobin, wherein the method comprises:

   measuring a measurement value Re correlating with a substrate concentration influenced by hemoglobin, and a hemoglobin concentration or a value correlating with the hemoglobin concentration; and
   wherein the substrate concentration is calculated by correcting the measurement value Re correlating with the substrate concentration based on the following Formulas 1 and 2:

$$\text{Formula 1}$$

$$\text{Corrected value} = 100 \times \text{Re}/\text{V}(\%),$$

   wherein

   V (%) represents a value with respect to plasma (a ratio of a measurement value influenced by hemoglobin with respect to a measurement value obtained by measuring plasma); and
   Re represents a measurement value correlating with a substrate concentration influenced by hemoglobin;

$$\text{Formula 2:}$$

$$\text{Value with respect to plasma V}(\%) = (\text{Vmax} \times \text{Re})/(\text{Km}+\text{Re})+\text{B},$$

   wherein

   Vmax represents a value obtained by subtracting an intercept (B) from a value (V) with respect to plasma which is maintained constant even when a measurement value influenced by hemoglobin increases;
   Km represents a value of Re at which a value V (%) with respect to plasma becomes Vmax/2+B; and

B represents a value (constant) of V (%) with respect to plasma when Re is 0.

2. The substrate concentration measurement method according to claim 1, wherein Vmax is determined based on a measurement result of the hemoglobin concentration or the value correlating with the hemoglobin concentration of the blood specimen.

3. The substrate concentration measurement method according to claim 2, wherein Vmax is determined based on a chromatogram obtainable by chromatography.

4. The substrate concentration measurement method according to claim 1, wherein the measurement value Re is measured by an enzyme electrode method.

5. The substrate concentration measurement method according to claim 4, wherein the measurement value Re is a voltage value or a current value.

6. A substrate concentration measurement apparatus (1) comprising:

a calculation means (5) that calculates a concentration of a substrate in a blood specimen containing hemoglobin and
a substrate measurement mechanism (3) that measures a value correlating with a substrate concentration influenced by hemoglobin, wherein
the substrate concentration measurement apparatus (1) further comprises a hemoglobin measurement mechanism (23) that measures a hemoglobin concentration or a value correlating with the hemoglobin concentration, and
the calculation means (5) is adapted to calculate the substrate concentration by using the measurement value Re in the substrate measurement mechanism (3) and the measurement value in the hemoglobin measurement mechanism (23);
wherein the calculation means is further adapted to calculate the substrate concentration by correcting the measurement value Re correlating with the substrate concentration based on the following Formulas 1 and 2:

$$\text{Formula 1:}$$

$$\text{Corrected value} = 100 \times Re/V(\%),$$

wherein

V (%) represents a value with respect to plasma (a ratio of a measurement value influenced by hemoglobin with respect to a measurement value obtained by measuring plasma); and
Re represents a measurement value correlating with a substrate concentration influenced by hemoglobin;

$$\text{Formula 2:}$$

$$\text{Value with respect to plasma } V(\%) = (Vmax \times Re)/(Km+Re)+B,$$

wherein

Vmax represents a value obtained by subtracting an intercept (B) from a value (V) with respect to plasma which is maintained constant even when a measurement value influenced by hemoglobin increases;
Km represents a value of Re at which a value V (%) with respect to plasma becomes Vmax/2+B; and
B represents a value (constant) of V (%) with respect to plasma when Re is 0.

7. The substrate concentration measurement apparatus (1) according to claim 6, wherein the calculation means (5) is adapted to determine Vmax by measuring the hemoglobin concentration or the value correlating with the hemoglobin concentration of the blood specimen.

8. The substrate concentration measurement apparatus (1) according to claim 7, wherein the hemoglobin measurement mechanism (23) is adapted to measure the hemoglobin concentration or the value correlating with the hemoglobin concentration as a chromatogram obtainable by chromatography.

9. The substrate concentration measurement apparatus (1) according to claim 6, wherein the substrate measurement mechanism (3) comprises an enzyme electrode (33) for obtaining the measurement value Re correlating with the substrate concentration influenced by hemoglobin.

10. The substrate concentration measurement apparatus (1) according to claim 9, wherein the measurement value Re is a current value outputted in the enzyme electrode (33) or a voltage value obtained by converting the current value.

**Patentansprüche**

1. Substratkonzentrationsmessverfahren zum Messen einer Konzentration eines Substrats in einer Hämoglobin enthaltenden Blutprobe, wobei das Verfahren umfasst:

Messen eines Messwerts Re, der mit einer durch Hämoglobin beeinflussten Substratkonzentration korreliert, und einer Hämoglobinkonzentration oder eines mit der Hämoglobinkonzentration korrelierenden Werts; und wobei die Substratkonzentration basierend auf den folgenden Formeln 1 und 2 durch Korrigieren des Messwerts Re berechnet wird, der mit der Substratkonzentration korreliert:

Formel 1

$$\text{Korrigierter Wert} = 100 \times Re/V(\%),$$

wobei

V(%) einen Wert in Bezug auf ein Plasma wiedergibt (ein Verhältnis eines durch Hämoglobin beeinflussten Messwerts in Bezug zu einem durch Messen von Plasma erhaltenen Messwerts); und
Re einen Messwert wiedergibt, der mit einer durch Hämoglobin beeinflussten Substratkonzentration korreliert;

Formel 2:

$$\text{Wert bezüglich Plasma } V(\%) = (Vmax \times Re)/(Km + Re) + B,$$

wobei

Vmax einen Wert wiedergibt der durch Abziehen eines Parameters (B) von einem Wert (V) in Bezug auf Plasma erhalten wird, der konstant gehalten wird, und zwar selbst dann, wenn ein durch Hämoglobin beeinflusster Messwert ansteigt;
Km einen Wert von Re wiedergibt, bei dem ein Wert V(%) in Bezug auf Plasma Vmax/2+B wird; und
B einen Wert (Konstante) von V(%) in Bezug auf Plasma wiedergibt, wenn Re null ist.

2. Substratkonzentrationsmessverfahren nach Anspruch 1, bei dem Vmax basierend auf einem Messergebnis der Hämoglobinkonzentration oder des mit der Hämoglobinkonzentration korrelierenden Werts der Blutprobe bestimmt wird.

3. Substratkonzentrationsmessverfahren nach Anspruch 2, bei dem Vmax basierend auf einem Chromatogramm bestimmt wird, das durch Chromatographie erhalten werden kann.

4. Substratkonzentrationsmessverfahren nach Anspruch 1, bei dem der Messwert Re durch ein Enzymelektrodenverfahren gemessen wird.

**5.** Substratkonzentrationsmessverfahren nach Anspruch 4, bei dem der Messwert Re ein Spannungswert oder ein Stromwert ist.

**6.** Substratkonzentrationsmessvorrichtung (1), mit:

einem Berechnungsmittel (5), das eine Konzentration eines Substrats in einer Blutprobe berechnet, die Hämoglobin enthält; und
einem Substratmechanismus (3), der einen Wert misst, der mit einer Substratkonzentration korreliert, die durch Hämoglobin beeinflusst wird, wobei
die Substratkonzentrationsmessvorrichtung (1) ferner einen Hämoglobinmessmechanismus (23) aufweist, der eine Hämoglobinkonzentration oder einen mit der Hämoglobinkonzentration korrelierenden Wert misst, und
das Berechnungsmittel (5) angepasst ist, die Substratkonzentration durch Verwenden des Messwerts Re bei dem Substratmessmechanismus (3) und dem Messwert bei dem Hämoglobinmessmechanismus (23) zu messen;
wobei das Berechnungsmittel ferner angepasst ist, die Substratkonzentration durch Korrigieren des Messwerts Re, der mit der Substratkonzentration korreliert, basierend auf den folgenden Formeln 1 und 2 zu berechnen:

Formel 1:

$$\text{Korrigierter Wert} = 100 \times \text{Re}/V(\%),$$

wobei

V(%) einen Wert in Bezug auf Plasma wiedergibt (ein Verhältnis eines durch Hämoglobin beeinflussten Messwerts in Bezug auf einen durch Messen von Plasma erhaltenen Messwert); und

Re einen Messwert wiedergibt, der mit einer durch Hämoglobin beeinflussten Substratkonzentration korreliert;

Formel 2:

$$\text{Wert bezüglich Plasma } V(\%) = (V_{max} \times \text{Re})/(K_m + \text{Re}) + B,$$

wobei
Vmax einen Wert wiedergibt, der durch Abziehen eines Parameters (B) von einem Wert (V) in Bezug auf Plasma erhalten wird, der konstant gehalten wird, und zwar selbst dann, wenn ein durch Hämoglobin beeinflusster Messwert ansteigt;
Km einen Re-Wert wiedergibt, bei dem ein Wert V(%) in Bezug auf Plasma Vmax/2+B wird; und
B einen Wert (Konstante) von V(%) in Bezug auf Plasma wiedergibt, wenn Re null ist.

**7.** Substratkonzentrationsmessvorrichtung (1) nach Anspruch 6, bei der das Berechnungsmittel (5) angepasst ist, Vmax durch Messen der Hämoglobinkonzentration oder des mit der Hämoglobinkonzentration korrelierenden Werts der Blutprobe zu bestimmen.

**8.** Substratkonzentrationsmessvorrichtung (1) nach Anspruch 7, bei welcher der Hämoglobinmessmechanismus (23) angepasst ist, die Hämoglobinkonzentration oder den mit der Hämoglobinkonzentration korrelierenden Wert als Chromatogramm zu messen, das durch Chromatographie erhalten werden kann.

**9.** Substratkonzentrationsmessvorrichtung (1) nach Anspruch 6, bei welcher der Substratmessmechanismus (3) eine Enzymelektrode (33) zum Erhalten des Messwerts Re aufweist, der mit der durch Hämoglobin beeinflussten Substratkonzentration korreliert.

**10.** Substratkonzentrationsmessvorrichtung (1) nach Anspruch 9, bei welcher der Messwert Re ein Stromwert ist, der bei der Enzymelektrode (33) ausgegeben wird, oder ein Spannungswert, der durch Umwandlung des Stromwerts erhalten wird.

**Revendications**

1. Procédé de mesure d'une concentration de substrat destiné à mesurer une concentration de substrat dans un échantillon sanguin qui contient de l'hémoglobine, le procédé comprenant :

   la mesure d'une valeur de mesure Re corrélée à une concentration de substrat influencée par l'hémoglobine et d'une concentration en hémoglobine ou d'une valeur corrélée à la concentration en hémoglobine ; et
   dans lequel la concentration de substrat est calculée en corrigeant la valeur de mesure Re corrélée à la concentration de substrat sur la base des formules 1 et 2 suivantes :

$$\text{Formule 1}$$

$$\text{Valeur corrigée} = 100 \times Re/V(\%),$$

   où

   V(%) représente une valeur par rapport au plasma (rapport entre une valeur de mesure influencée par l'hémoglobine et une valeur de mesure obtenue en mesurant le plasma) ; et
   Re représente une valeur de mesure corrélée à une concentration de substrat influencée par l'hémoglobine ;

$$\text{Formule 2}$$

$$\text{Valeur par rapport au plasma } V(\%) = (Vmax \times Re)/(Km + Re) + B,$$

   où

   Vmax représente une valeur obtenue en soustrayant un intercept (B) d'une valeur (V) par rapport au plasma qui est maintenu constant même lorsqu'une valeur de mesure influencée par l'hémoglobine augmente ;
   Km représente une valeur de Re à laquelle une valeur V(%) par rapport au plasma devient Vmax/2 + B ; et
   B représente une valeur (constante) de V(%) par rapport au plasma lorsque Re est égal à 0.

2. Procédé de mesure d'une concentration de substrat selon la revendication 1, dans lequel Vmax est déterminé sur la base d'un résultat de mesure de la concentration en hémoglobine ou de la valeur corrélée à la concentration en hémoglobine de l'échantillon sanguin.

3. Procédé de mesure d'une concentration de substrat selon la revendication 2, dans lequel Vmax est déterminé sur la base d'un chromatogramme qui peut être obtenu par chromatographie.

4. Procédé de mesure d'une concentration de substrat selon la revendication 1, dans lequel la valeur de mesure Re est mesurée par un procédé d'électrode enzymatique.

5. Procédé de mesure d'une concentration de substrat selon la revendication 4, dans lequel la valeur de mesure Re est une valeur de tension ou une valeur de courant.

6. Appareil de mesure d'une concentration de substrat (1) qui comprend :

   un moyen de calcul (5) qui calcule une concentration de substrat dans un échantillon sanguin qui contient de l'hémoglobine ; et
   un mécanisme de mesure de substrat (3) qui mesure une valeur corrélée à une concentration de substrat influencée par l'hémoglobine, dans lequel
   l'appareil de mesure d'une concentration de substrat (1) comprend en outre un mécanisme de mesure de l'hémoglobine (23) qui mesure une concentration en hémoglobine ou une valeur corrélée à la concentration en hémoglobine ; et

le moyen de calcul (5) est adapté pour calculer la concentration de substrat à l'aide de la valeur de mesure Re dans le mécanisme de mesure de substrat (3) et de la valeur de mesure dans le mécanisme de mesure de l'hémoglobine (23) ;

dans lequel le moyen de calcul est en outre adapté pour calculer la concentration de substrat en corrigeant la valeur de mesure Re corrélée à la concentration de substrat sur la base des formules 1 et 2 suivantes :

## Formule 1

$$\text{Valeur corrigée} = 100 \times Re/V(\%),$$

où

V(%) représente une valeur par rapport au plasma (un rapport entre une valeur de mesure influencée par l'hémoglobine et une valeur de mesure obtenue en mesurant le plasma) ; et

Re représente une valeur de mesure corrélée à une concentration de substrat influencée par l'hémoglobine ;

## Formule 2

$$\text{Valeur par rapport au plasma } V(\%) = (Vmax \times Re)/(Km + Re) + B,$$

où

Vmax représente une valeur obtenue en soustrayant un intercept (B) d'une valeur (V) par rapport au plasma qui est maintenu constant même lorsqu'une valeur de mesure influencée par l'hémoglobine augmente ;

Km représente une valeur de Re à laquelle une valeur V(%) par rapport au plasma devient Vmax/2 + B ; et

B représente une valeur (constante) de V(%) par rapport au plasma lorsque Re est égal à 0.

**7.** Appareil de mesure d'une concentration de substrat (1) selon la revendication 6, dans lequel le moyen de calcul (5) est adapté pour déterminer Vmax en mesurant la concentration en hémoglobine ou la valeur corrélée à la concentration en hémoglobine de l'échantillon sanguin.

**8.** Appareil de mesure d'une concentration de substrat (1) selon la revendication 7, dans lequel le mécanisme de mesure de l'hémoglobine (23) est adapté pour mesurer la concentration en hémoglobine ou la valeur corrélée à la concentration en hémoglobine sous forme d'un chromatogramme qui peut être obtenu par chromatographie.

**9.** Appareil de mesure d'une concentration de substrat (1) selon la revendication 6, dans lequel le mécanisme de mesure du substrat (3) comprend une électrode enzymatique (33) destinée à fournir la valeur de mesure Re par corrélation avec la concentration de substrat influencée par l'hémoglobine.

**10.** Appareil de mesure d'une concentration de substrat (1) selon la revendication 9, dans lequel la valeur de mesure Re est une valeur de courant fournie à l'électrode enzymatique (33) ou une valeur de tension obtenue en convertissant la valeur de courant.

# FIG.1

EP 2 151 682 B1

# FIG.2

# FIG.3

# FIG.4

# FIG.5

# FIG.6

TOTAL HEMOGLOBIN AMOUNT

# FIG.7

Hct45%[WHOLE BLOOD]

# FIG.8

Hct35%[LOW Htc WHOLE BLOOD]

# FIG.9

Hct45%[HIGH Htc WHOLE BLOOD]

# FIG.10

**ACCURACY OF Htc & Hb INFLUENCE CORRECTION**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 9033533 A
- JP 9318634 A
- WO 2005098424 A